# EUROPEAN PATENT APPLICATION

(11) **EP 4 544 982 A1**
(43) Date of publication of application: **30.04.2025**
(21) Application number: 23830194.9
(22) Date of filing: 26.06.2023
(51) Int. Cl.: A61B 1/005, A61B 1/01

(54) **CONTROLLABLE BENDING MEDICAL INSTRUMENT**

(30) Priority: 27.06.2022 CN 202210744085
(71) Applicant: Zhejiang Easyscopy Medical Co., Ltd., Jiaxing, Zhejiang 314199 (CN)
(72) Inventor: XIE, Songlin, Jiaxing, Zhejiang 314199 (CN); NIU, Quanyun, Jiaxing, Zhejiang 314199 (CN); HU, Xiaobo, Jiaxing, Zhejiang 314199 (CN); NIE, Honglin, Jiaxing, Zhejiang 314199 (CN)
(74) Representative: Daub, Thomas
(86) International application number: PCT/CN2023/102455
(87) International publication number: WO 2024/002017

(57) **Abstract**

Provided is a controllable bendable medical instrument (1), including a controllable bendable tube (30), a traction assembly (50, 60), and a rotating disc (10). One end of the traction assembly (50, 60) is fixedly connected to the rotating disc (10), and the other end of the traction assembly (50, 60) is connected to the controllable bendable tube (30). The traction assembly (50, 60) is pulled or loosened by rotating the rotating disc (10) to drive the controllable bendable tube (30) in the controllable bendable medical instrument (1) to bend. The rotating disc (10) is provided with adjusting bosses (12, 15). When the traction assembly (50, 60) is assembled, the tightness of the traction assembly (50, 60) is adjusted by adjusting a winding path of the traction assembly (50, 60) around the adjusting bosses (12, 15), thus meeting the initial assembly requirement.

## Description

### FIELD

The present application relates to the field of medical devices, and particularly to a controllable bendable medical instrument.

### BACKGROUND

A controllable bendable tube (i.e., flexible tube) is an important element of an endoscope and a core-associated element for controlling the direction of a visual component, and its assembly effect directly determines the performance of a product. The tightness of the pull wire is an important index in the assembly of the controllable bendable tube, and an excessively tight pull wire will cause the controllable bendable tube to be initially bent, which will affect the use of the controllable bendable tube. If the pull wire is too loose, the maximum bending of the controllable bendable tube is not enough during use, which will affect the visual control effect.

### SUMMARY

In order to solve the problem of how to adjust the tightness of the pull wire when assembling the pull wire of an endoscope, a primary object of the present application is to provide a controllable bendable medical instrument, which may adjust the tightness of the pull wire.

In a first aspect, the present disclosure provides a controllable bendable medical instrument, including a controllable bendable tube, a traction assembly, and a rotating disc, wherein one end of the traction assembly is fixedly connected to the rotating disc, and the other end of the traction assembly is connected to the controllable bendable tube; the traction assembly is pulled or loosened by rotating the rotating disc to drive the controllable bendable tube in the controllable bendable medical instrument to bend; the rotating disc is provided with adjusting bosses, wherein during assembly of the traction assembly, the tightness of the traction assembly is adjusted by adjusting a winding path of the traction assembly around the adjusting bosses.

Optionally, the rotating disc is provided with a first fixing boss, and at least a part of the traction assembly is fixedly mounted to the first fixing boss.

Optionally, the traction assembly includes a first pull wire, and the adjusting bosses include a first adjusting boss; one end of the first pull wire is fixedly connected to the first fixing boss, and the other end of the first pull wire is connected to the controllable bendable tube; the tightness of the first pull wire is adjusted by adjusting a winding path of the first pull wire around the first adjusting boss.

Optionally, the traction assembly further includes a second pull wire; one end of the second pull wire is fixedly connected to the rotating disc, and the other end of the second pull wire is connected to the controllable bendable tube.

Optionally, the rotating disc is provided with a second fixing boss, and the adjusting bosses include a second adjusting boss; one end of the second pull wire is fixedly connected to the second fixing boss, and the other end of the second pull wire is connected to the controllable bendable tube; the tightness of the second pull wire is adjusted by adjusting a winding path of the second pull wire around the second adjusting boss.

Optionally, the rotating disc further includes a plurality of the first adjusting bosses and a plurality of the second adjusting bosses.

Optionally, the first pull wire is wound around one or more of the first adjusting bosses, or the first pull wire is not wound around one or more of the first adjusting bosses; and
the second pull wire is wound around one or more of the second adjusting bosses, or the second pull wire is not wound around one or more of the second adjusting bosses.

Optionally, the first fixing boss, the first adjusting boss, the second fixing boss, and the second adjusting boss are of cylindrical boss structures formed on a surface of the rotating disc.

Optionally, the rotating disc further includes a first steering boss and/or a second steering boss; the first steering boss is configured to change a winding direction of the first pull wire, and the second steering boss is configured to change a winding direction of the second pull wire.

Optionally, the rotating disc is formed with a first through hole and a second through hole; the first pull wire passes through the first through hole to limit a position of the first pull wire, and the second pull wire passes through the second through hole to limit a position of the second pull wire.

Optionally, one end of the first pull wire is first wound around the first fixing boss and then wound around the first adjusting boss and the first steering boss, and finally, the other end of the first pull wire passes through the first through hole and is connected to the controllable bendable tube.

Optionally, the controllable bendable medical instrument further includes a damping member; the damping member provides resistance to the rotation of the rotating disc.

Optionally, a handle is provided with a housing, and the damping member is located between the rotating disc and the housing; the damping member providesresistance to the rotation of the rotating disc relative to the housing.

Optionally, the controllable bendable medical instrument further includes an operating member; the operating member is connected to the rotating disc, and the rotating disc is driven to rotate by manipulating the operating member.

Optionally, the rotating disc is a rotating wheel.

According to the controllable bendable medical instrument of the above-mentioned solutions, the traction assembly is mounted and fixed through the adjusting assemblies so that the extension path of the traction assembly changes, thereby adjusting the tightness of the traction assembly. Therefore, the controllable bendable tube may be bent to a preset angle unhinderedly.

### BRIEF DESCRIPTION OF THE DRAWINGS

FIG. 1 is a schematic structural diagram of a controllable bendable medical instrument according to one implementation of the present disclosure.
FIG. 2a is a schematic structural diagram of a rotating disc according to one implementation of the present disclosure.
FIG. 2b is a schematic structural diagram of the rotating disc of FIG. 2a in cooperation with a part of a first pull wire and a part of a second pull wire.
FIG. 3a is a schematic structural diagram of a rotating disc in cooperation with a first pull wire, a second pull wire, and a controllable bendable tube at a certain angle (the rotating disc is provided with first steering bosses and second steering bosses).
FIG. 3b is a schematic structural diagram of a rotating disc in cooperation with a first pull wire, a second pull wire, and a controllable bendable tube at another angle.
FIG. 3c is a schematic structural diagram of a rotating disc in cooperation with a first pull wire, a second pull wire, and a controllable bendable tube at a certain angle (the rotating disc is not provided with a first steering boss and a second steering boss).
FIG. 3d is a schematic structural diagram of a rotating disc in cooperation with a first pull wire and a controllable bendable tube at a certain angle.
FIG. 4a is a schematic structural diagram of a rotating disc in cooperation with a part of a first pull wire (the first pull wire is connected to one first adjusting boss).
FIG. 4b is a schematic structural diagram of a rotating disc in cooperation with a part of a first pull wire (the first pull wire is connected to a certain first adjusting boss).
FIG. 4c is a schematic structural diagram of a rotating disc in cooperation with a part of a first pull wire (the first pull wire is connected to another first adjusting boss).
FIG. 4d is a schematic structural diagram of a rotating disc in cooperation with a part of a first pull wire (the first pull wire is connected to a further first adjusting boss).
FIG. 4e is a schematic structural diagram of a rotating disc in cooperation with a part of a first pull wire (the first pull wire is not connected to the first adjusting boss).
FIG. 5 is a schematic structural diagram of a controllable bendable medical instrument.

### DETAILED DESCRIPTION

In order to further clarify the technical means and effects of the present application for achieving the intended purpose, a detailed description of specific implementations, structures, features, and effects thereof according to the present application is provided below with reference to the accompanying drawings and preferred embodiments.

In the description of the present application, it should be noted that orientation or positional relationships indicated by the terms "center", "upper", "lower", "left", "right", "vertical", "horizontal", "inner", "outer", and the like are based on the orientation or positional relationships shown in the drawings, are intended only to facilitate and simplify the description of the present application, and are not intended to indicate or imply that the apparatus or element referred to must have a particular orientation or be constructed and operated in a particular orientation, and therefore are not to be construed as limitations of the present application. In addition, the terms "first", "second", and "third" are used for descriptive purposes only and are not to be construed as indicating or implying relative importance.

In the description of the present application, it should be noted that, unless otherwise explicitly stated or limited, the terms "mounted", "communicated", and "connected" are to be construed broadly, such as fixedly connected, detachably connected, integrally connected, mechanically connected, electrically connected, directly connected, indirectly connected through an intermediate medium, and communicated between two elements. The term "communicated" should also be construed broadly, such as directly communicated or indirectly communicated through an intermediate medium. The specific meanings of the above-mentioned terms in the present application may be understood by a person skilled in the art according to specific circumstances.

In addition, the technical features involved in different implementations of the present application described below may be combined with each other as long as they do not conflict with each other.

As used in the present disclosure and the appended claims, the singular forms "a/an", "the", and "this" are intended to include the plural forms as well, unless the context clearly indicates otherwise. It should also be understood that the term "and/or" as used herein refers to and encompasses any or all possible combinations of one or more of the associated listed items.

As used in the present disclosure and the appended claims, the terms "a plurality of" and "multiple" refer to two or more unless specifically stated otherwise.

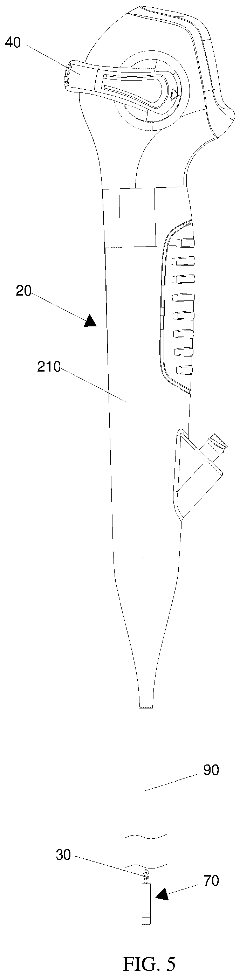

It should be noted that a controllable bendable tube 30 includes a plurality of interconnected bendable tube units (also referred to as units of snake-like tube), and adjacent bendable tube units may be rotated relative to each other, thereby allowing the entire controllable bendable tube 30 to be bent. Structures of the controllable bendable tube 30 will not be described in the present disclosure due to the variety. Specifically, as shown in FIG. 5, a controllable bendable medical instrument 1 includes an insertion portion 70, and the insertion portion 70 includes a controllable bendable tube 30 and an insertion tube 90. A proximal end of the insertion tube 90 is connected to a handle 20, and a distal end of the insertion tube 90 is connected to the controllable bendable tube 30. More specifically, the distal end of the insertion tube 90 is connected to a bendable tube unit at the most proximal end of the controllable bendable tube 30. A bendable tube unit at the most distal end of the controllable bendable tube 30 is connected to a precursor portion (also referred to as a head end portion), and the precursor portion is configured to mount an optical sensor and other structures. One end of the traction assembly 50, 60 is mounted on the rotating disc 10, and the other end of the traction assembly 50, 60 passes through the inside of the insertion tube 90 and the controllable bendable tube 30 and is connected to the bendable tube unit at the most distal end. The manner in which the traction assembly 50, 60 is fixedly mounted on the rotating disc 10 includes, but is not limited to, glue bonding, bolting, riveting, heat fusing, and screwing. It should be understood that the above description is intended only to better illustrate the connection of the various structures in the controllable bendable medical instrument 1, and does not limit the structure of the controllable bendable medical instrument 1. In addition, the proximal end refers to an end close to the user (i.e., an end where the handle is located), and the distal end refers to an end away from the user (mainly refers to an end where the precursor portion is located).

Due to the issue of errors during the production and assembly of parts, the tightness of the traction assembly 50, 60 cannot reach the requirements, and thus the controllable bendable tube 30 cannot be normally bent or bent to a right position. Therefore, the present disclosure provides a controllable bendable medical instrument 1, including a controllable bendable tube 30, a traction assembly 50, 60, and a rotating disc 10. One end of the traction assembly 50, 60 is fixedly connected to the rotating disc 10, and the other end of the traction assembly 50, 60 is connected to the controllable bendable tube 30. The traction assembly 50, 60 is pulled or loosened by rotating the rotating disc 10 to drive the controllable bendable tube 30 in the controllable bendable medical instrument 1 to bend. The rotating disc 10 is provided with adjusting bosses 12, 15. During assembly of the traction assembly 50, 60, the tightness of the traction assembly 50, 60 is adjusted by adjusting a winding path of the traction assembly 50, 60 around the adjusting bosses 12, 15. Specifically, the extension path of the traction assembly 50, 60 is adjusted by winding the traction assembly 50, 60 around the adjusting bosses 12, 15, thereby adjusting the tightness of the traction assembly 50, 60. The traction assembly 50, 60, in addition to being a steel wire, may be made of any suitable material, such as metal, metal alloy, carbon fiber, glass fiber, polymer wire, natural fiber, and/or any other suitable material or combination of materials. Assembling the traction assembly 50, 60 specifically means mounting the traction assembly 50, 60 on the adjusting bosses 12, 15, the rotating disc 10, and the controllable bendable tube 30.

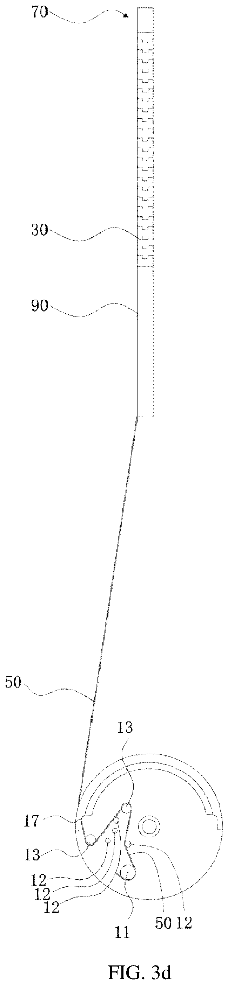

Specifically, as shown in FIG. 3d, the rotating disc 10 is provided with a first fixing boss 11, and at least a part of the traction assembly 50, 60 is fixedly mounted to the first fixing boss 11. More specifically, the traction assembly 50, 60 includes a first pull wire 50, and the adjusting bosses 12, 15 include first adjusting bosses 12. One end of the first pull wire 50 is fixedly connected to the first fixing boss 11, and the other end of the first pull wire 50 is connected to the controllable bendable tube 30. The tightness of the first pull wire 50 is adjusted by adjusting a winding path of the first pull wire 50 around the first adjusting bosses 12. It should be understood that in some implementations, as shown in FIG. 3d, only the first fixing boss 11 and the first adjusting bosses 12 are provided on the rotating disc 10, it is not necessary to provide the second fixing boss 14 and the second adjusting boss 15. Further, in order to facilitate fixing of the first pull wire 50, the first fixing boss 11 is provided on the rotating disc 10. However, in other implementations, the first pull wire 50 may not be fixedly mounted on the first fixing boss 11, but may be fixedly mounted on other positions of the rotating disc 10. Optionally, the traction assembly 50, 60 further includes a second pull wire 60. One end of the second pull wire 60 is fixedly connected to the rotating disc 10, and the other end of the second pull wire 60 is connected to the controllable bendable tube 30.

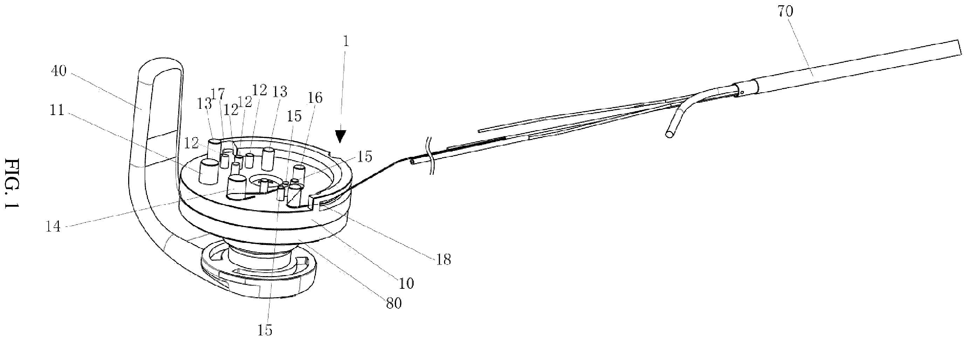

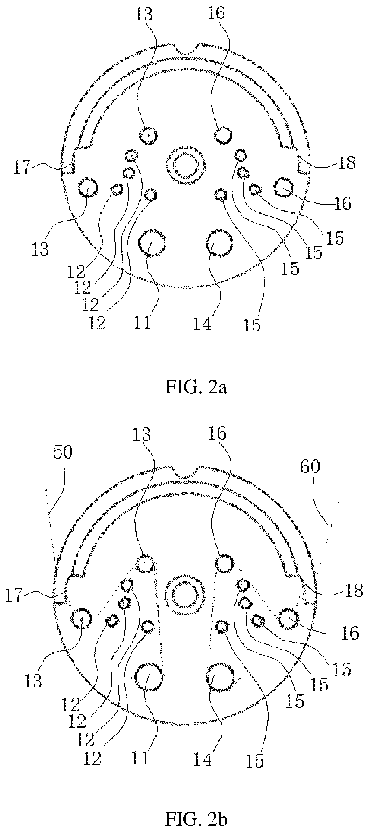

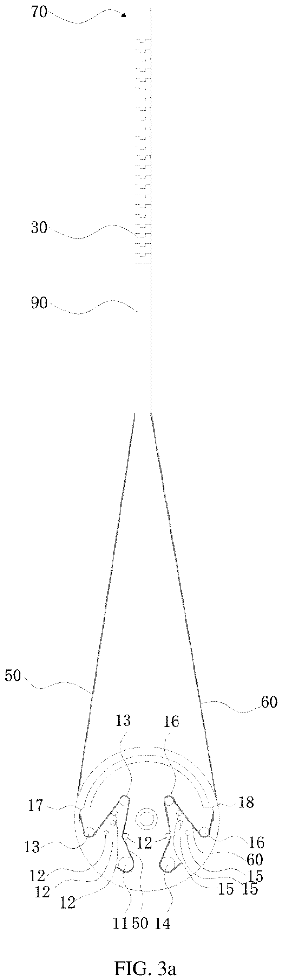

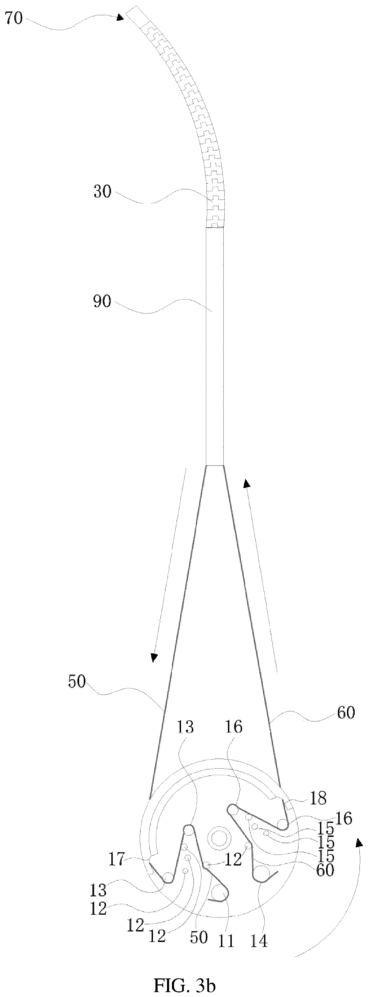

Optionally, as shown in FIGS. 1, 2a, and 2b, the rotating disc 10 is provided with a second fixing boss 14, and the adjusting bosses 12, 15 include second adjusting bosses 15. One end of the second pull wire 60 is fixedly connected to the second fixing boss 14, and the other end of the second pull wire 60 is connected to the controllable bendable tube 30. The tightness of the second pull wire 60 is adjusted by adjusting a winding path of the second pull wire 60 around the second adjusting bosses 15. The rotating disc 10 is rotated to pull or loosen the first pull wire 50 and the second pull wire 60 so that the controllable bendable tube 30 is bent. For example, the rotating disc 10 may initially be in the position shown in FIG. 3a, and when the rotating disc 10 is rotated counterclockwise, the first pull wire 50 is tightened so that the other end of the first pull wire 50 pulls one side of the controllable bendable tube 30. Meanwhile, the second pull wire 60 is loosened to bend the controllable bendable tube 30 to the side connected to the first pull wire 50, as shown in FIG. 3b. Similarly, when the rotating disc 10 is rotated clockwise, the first pull wire 50 is loosened while the second pull wire 60 is tightened so that the other end of the second pull wire 60 pulls the other side of the controllable bendable tube 30 to bend the controllable bendable tube 30 to the other side connected to the second pull wire 60.

In addition, the first pull wire 50 may be fixedly connected to the first fixing boss 11 by glue bonding, bolting, riveting, heat fusing, screwing, etc. The second pull wire 60 may be fixedly connected to the second fixing boss 14 by glue bonding, bolting, riveting, heat fusing, screwing, etc.

In the above-mentioned implementation, the traction assembly 50, 60 includes the first pull wire 50 and the second pull wire 60, the rotating disc 10 is provided with the first fixing boss 11 and the second fixing boss 14, and the adjusting bosses 12, 15 include the first adjusting bosses 12 and the second adjusting bosses 15. It should be understood that, in the above-mentioned implementation, while only one first pull wire 50 and one second pull wire 60 are included, the left-and-right or up-and-down bending of the controllable bendable tube 30 may be controlled. However, in some implementations, two sets of pull wires may be included, i.e., a first pull wire set and a second pull wire set. Each pull wire set includes one first pull wire 50 and one second pull wire 60. The first pull wire set is configured to drive the left-and-right bending of the controllable bendable tube 30, and the second pull wire set is configured to drive the up-and-down bending of the controllable bendable tube 30. In addition, the first pull wire 50 may or may not be wound more than one turn on the first adjusting boss 12, and the number of winding turns specifically depends on the actual situation and is not limited by the present disclosure. Similarly, the second pull wire 60 may or may be wound more than one turn on the second adjusting boss 15.

Optionally, as shown in FIGS. 2a and 2b, the rotating disc 10 further includes first steering bosses 13 and/or second steering bosses 16. The first steering boss 13 is configured to change a winding direction of the first pull wire 50, and the second steering boss 16 is configured to change a winding direction of the second pull wire 60. The first steering boss 13 and the second steering boss 16 share the pulling forces of the first fixing boss 11 and the second fixing boss 14, respectively. Specifically, the winding directions of the first pull wire 50 and the second pull wire 60 are changed so that a part of the pulling force along the extension direction of the first pull wire 50 and the second pull wire 60 is divided to laterally act on the first pull wire 50 and the second pull wire 60, thereby reducing the possibility of the first pull wire 50 and the second pull wire 60 breaking under excessive pulling force. It should be understood that the winding direction refers to the extension direction or winding direction of the pull wire. As shown in FIG. 2b, the first pull wire 50 passes through two first steering bosses 13. The winding direction of the first pull wire 13 changes each time a first steering boss 13 is passed.

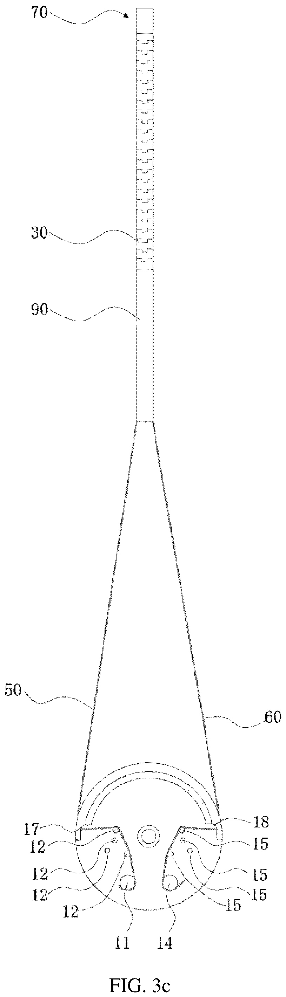

In addition, the number of the first steering bosses 13 and the second steering bosses 16 may be adjusted according to circumstances, such as 1, 2, 3, or more, and is not specifically limited by the present disclosure. Furthermore, in some implementations, as shown in FIG. 3c, the rotating disc 10 is not necessarily provided with a first steering boss 13 and a second steering boss 16.

Optionally, as shown in FIG. 1, the rotating disc 10 is formed with a first through hole 17 and a second through hole 18, and the first pull wire 50 passes through the first through hole 17. A part of the first pull wire 50 located at the first through hole 17 is closer to the other end of the first pull wire 50 (i.e., the end connected to the controllable bendable tube 30) along the extension direction of the first pull wire 50 than a part of the first pull wire 50 connected to the first adjusting boss 12. In addition, the part of the first pull wire 50 located at the first through hole 17 is closer to the other end of the first pull wire 50 along the extension direction of the first pull wire 50 than a part of the first pull wire 50 connected to the first steering boss 13. Specifically, one end of the first pull wire 50 is first wound around the first fixing boss 11 and then wound around the first adjusting boss 12 and the first steering boss 13, and finally, the other end of the first pull wire 50 passes through the first through hole 17 and is connected to the controllable bendable tube 30. The purpose of this design is to limit the position of the first pull wire 50 after entering the rotating disc 10 and to avoid that the position of the first pull wire 50 on the rotating disc 10 changes during the rotation of the rotating disc 10 so that the winding directions of the first pull wire 50 on the first steering boss 13 and the first adjusting boss 12 change.

Likewise, the second pull wire 60 passes through the second through hole 18. In addition, a part of the second pull wire 60 located at the second through hole 18 is closer to the other end of the second pull wire 60 along the extension direction of the second pull wire 60 than a part of the second pull wire 60 connected to the second adjusting boss 15. Furthermore, the part of the second pull wire 60 located at the second through hole 18 is closer to the other end of the second pull wire 60 along the extension direction of the second pull wire 60 than a part of the second pull wire 60 connected to the second steering boss 16. Specifically, one end of the second pull wire 60 is first wound around the second fixing boss 14 and then wound around the second adjusting boss 15 and the second steering boss 16, and finally, the other end of the second pull wire 60 passes through the second through hole 18 and is connected to the controllable bendable tube 30. The above-mentioned design is also intended to avoid large changes in the position of the second pull wire 60 on the rotating disc 10 during the rotation of the rotating disc 10.

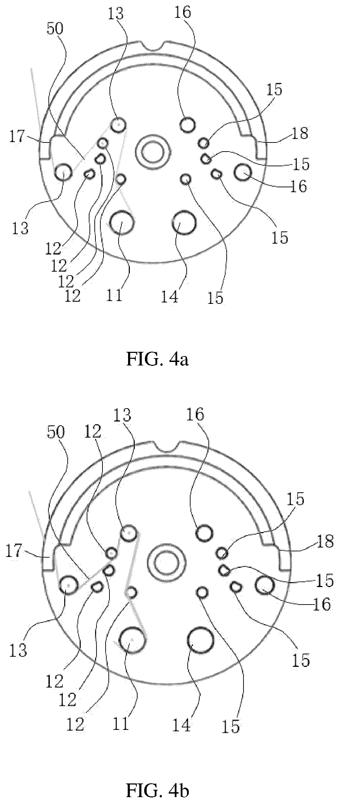

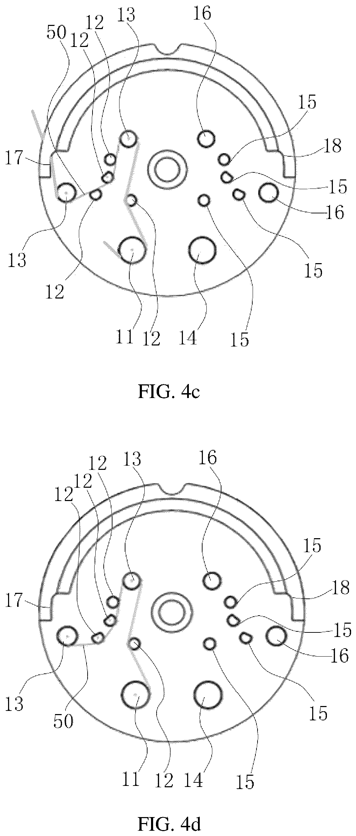

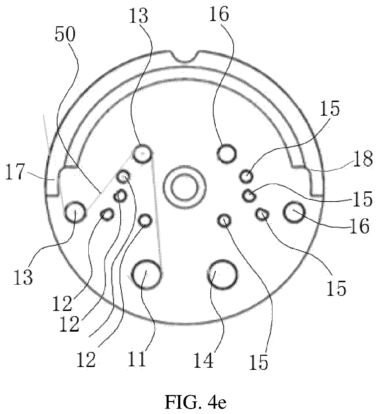

In order to increase the adjustable gear of the controllable bendable medical instrument 1 and to allow the factory to better adjust the tightness of the first pull wire 50 and the second pull wire 60, in some implementations, as shown in FIGS. 2a and 2b, the rotating disc 10 further includes a plurality of first adjusting bosses 12 and a plurality of second adjusting bosses 15. Specifically, the factory may select the first adjusting boss 12 to which the first pull wire 50 is fixedly connected according to circumstances. As shown in FIG. 4a, the first pull wire 50 may be wound around only one of the first adjusting bosses 12. If it is desired to tighten the first pull wire 50, the first pull wire 50 may be wound around different first adjusting bosses 12 to adjust the position and tightness of the first pull wire 50, as specifically shown in FIGS. 4b, 4c, and 4d. If it is desired to loosen the first pull wire 50, the first pull wire 50 may not be wound around any first adjusting boss 12, as specifically shown in FIG. 4e.

It should be understood that the first pull wire 50 may be wound around one or more of the first adjusting bosses 12 according to circumstances to adjust the tightness of the first pull wire 50. Similarly, the factory may also select the second pull wire 60 to wind around the second adjusting boss 15 according to circumstances so as to adjust the tightness of the second pull wire 60. In addition, the number of the first adjusting bosses 12 and the second adjusting bosses 15 is not limited to FIGS. 2a and 2b, and may be one, two, three, and four or more.

It should be understood that the manner in which the tightness of the traction assembly is adjusted through the first adjusting boss 12 and the second adjusting boss 15 is mainly during the assembly of the controllable bendable medical instrument 1. The factory selects the first adjusting boss 12 and the second adjusting boss 15 around which the first pull wire 50 and the second pull wire 60 are wound, respectively, according to the tightness of the first pull wire 50 and the second pull wire 60 during debugging so that the controllable bendable tube 30 may be bent normally. When the user uses the controllable bendable medical instrument 1, it is impossible to change the first pull wire 50 to wind around the first adjusting boss 12 and change the second pull wire 60 to wind around the second adjusting boss 15 by rotating the rotating disc 10.

In some embodiments, the controllable bendable medical instrument 1 further includes an operating member 40. The operating member 40 is connected to the rotating disc 10, and the rotating disc 10 is driven to rotate by manipulating the operating member 40. Specifically, the operating member 40 is an operating rod, and the operating rod is located outside a housing 210. However, the controllable bendable medical instrument 1 is located inside the housing 210, which facilitates the user to manipulate the rotation of the rotating disc 10 through the operating rod outside the housing 210, thereby controlling the bending of the controllable bendable tube 30. It should be understood that while in some implementations, the operating member 40 is an operating rod, in other implementations, the operating member 40 may be a hand lever, a knob, or the like, and is not limited by the present disclosure.

Referring to FIGS. 1 and 5, the controllable bendable medical instrument 1 further includes a handle 20 and an insertion tube 90. The rotating disc 10 is mounted on the handle 20. A proximal end of the insertion tube 90 is connected to the handle 20, and a distal end of the insertion tube 90 is connected to the controllable bendable tube 30.

Optionally, the controllable bendable medical instrument 1 further includes a damping member 80. The damping member 80 provides a resistance to the rotation of the rotating disc 10.

Optionally, the handle 20 is provided with the housing 210, and the damping member 80 is located between the rotating disc 10 and the housing 210. The damping member 80 provides a resistance to the rotation of the rotating disc 10 relative to the housing 210.

Specifically, the damping member 80 is a damping pad. Due to the action of the damping member 80, a certain force is required to rotate the rotating disc 10, allowing the rotating disc 10 to stop at any position within its rotation travel, thereby facilitating the user to control the bending degree of the controllable bendable tube 30 by rotating the rotating disc 10.

Optionally, the rotating disc 10 is a rotating wheel. In some implementations, it may also be other structures, and the present disclosure is not limited thereto.

It should be understood that the first fixing boss 11, the first adjusting boss 12, the second fixing boss 14, and the second adjusting boss 15 are of cylindrical boss structures formed on a surface of the rotating disc 10. In some embodiments, the first fixing boss 11, the first adjusting boss 12, the second fixing boss 14, and the second adjusting boss 15 may be fixedly connected to the rotating disc 10 by glue bonding, heat fusing, ultrasonic welding, etc.

The above-mentioned implementations are merely preferred implementations of the present application and are not intended to limit the scope of the present application. Any insubstantial changes and substitutions made by a person skilled in the art based on the present application fall within the scope of the present application.

## Claims

1. A controllable bendable medical instrument, comprising a controllable bendable tube, a traction assembly, and a rotating disc, wherein one end of the traction assembly is fixedly connected to the rotating disc, and the other end of the traction assembly is connected to the controllable bendable tube; the traction assembly is pulled or loosened by rotating the rotating disc to drive the controllable bendable tube in the controllable bendable medical instrument to bend;
**characterized in that**,
the rotating disc is provided with adjusting bosses,
wherein during assembly of the traction assembly, the tightness of the traction assembly is adjusted by adjusting a winding path of the traction assembly around the adjusting bosses.

2. The controllable bendable medical instrument according to claim 1, **characterized in that**, wherein the rotating disc is provided with a first fixing boss, and at least a part of the traction assembly is fixedly mounted to the first fixing boss.

3. The controllable bendable medical instrument according to any one of the preceding claims, **characterized in that**, wherein the traction assembly comprises a first pull wire, and the adjusting bosses comprise a first adjusting boss; one end of the first pull wire is fixedly connected to the first fixing boss, and the other end of the first pull wire is connected to the controllable bendable tube; the tightness of the first pull wire is adjusted by adjusting a winding path of the first pull wire around the first adjusting boss.

4. The controllable bendable medical instrument according to any one of the preceding claims, **characterized in that**, wherein the traction assembly further comprises a second pull wire; one end of the second pull wire is fixedly connected to the rotating disc, and the other end of the second pull wire is connected to the controllable bendable tube.

5. The controllable bendable medical instrument according to any one of the preceding claims, **characterized in that**, wherein the rotating disc is provided with a second fixing boss, and the adjusting bosses comprise a second adjusting boss; one end of the second pull wire is fixedly connected to the second fixing boss; the tightness of the second pull wire is adjusted by adjusting a winding path of the second pull wire around the second adjusting boss.

6. The controllable bendable medical instrument according to any one of the preceding claims, **characterized in that**, wherein the rotating disc further comprises a plurality of the first adjusting bosses and a plurality of the second adjusting bosses.

7. The controllable bendable medical instrument according to any one of the preceding claims, **characterized in that**, wherein the first pull wire is wound around one or more of the first adjusting bosses, or the first pull wire is not wound around one or more of the first adjusting bosses; and
the second pull wire is wound around one or more of the second adjusting bosses, or the second pull wire is not wound around one or more of the second adjusting bosses.

8. The controllable bendable medical instrument according to any one of the preceding claims, **characterized in that**, wherein the first fixing boss, the first adjusting boss, the second fixing boss, and the second adjusting boss are of cylindrical boss structures formed on a surface of the rotating disc.

9. The controllable bendable medical instrument according to any one of the preceding claims, **characterized in that**, wherein the rotating disc further comprises a first steering boss and/or a second steering boss; the first steering boss is configured to change a winding direction of the first pull wire, and the second steering boss is configured to change a winding direction of the second pull wire.

10. The controllable bendable medical instrument according to any one of the preceding claims, **characterized in that**, wherein the rotating disc is formed with a first through hole and a second through hole; the first pull wire passes through the first through hole to limit a position of the first pull wire, and the second pull wire passes through the second through hole to limit a position of the second pull wire.

11. The controllable bendable medical instrument according to any one of the preceding claims, **characterized in that**, wherein one end of the first pull wire is first wound around the first fixing boss and then wound around the first adjusting boss and the first steering boss, and finally, the other end of the first pull wire passes through the first through hole and is connected to the controllable bendable tube.

12. The controllable bendable medical instrument according to any one of the preceding claims, **characterized by**, further comprising a damping member, wherein the damping member provides resistance to the rotation of the rotating disc.

13. The controllable bendable medical instrument according to any one of the preceding claims, **characterized in that**, wherein a handle is provided with a housing, and the damping member is located between the rotating disc and the housing; the damping member provides resistance to the rotation of the rotating disc relative to the housing.

14. The controllable bendable medical instrument according to any one of the preceding claims, **characterized by**, further comprising an operating member, wherein the operating member is connected to the rotating disc, and the rotating disc is driven to rotate by manipulating the operating member.

15. The controllable bendable medical instrument according to any one of the preceding claims, **characterized in that**, wherein the rotating disc is a rotating wheel.
